# EUROPEAN PATENT APPLICATION

(11) **EP 4 298 902 A1**
(43) Date of publication of application: **03.01.2024**
(21) Application number: 22803822.0
(22) Date of filing: 10.05.2022
(51) Int. Cl.: A01K 67/027, C07K 16/06, C12N 15/85, C12N 15/09

(54) **PREPARATION METHOD FOR ANTIBODY CARRYING UNIVERSAL FIXED-POINT COUPLING INTERFACE BASED ON GENETICALLY MODIFIED VERTEBRATE**

(30) Priority: 18.05.2021 CN 202110539159
(71) Applicant: Unogen Biotechnology Co., Ltd, Suzhou, Jiangsu 215123 (CN)
(72) Inventor: CHEN, Tao, Suzhou, Jiangsu 215123 (CN); YUE, Bin, Suzhou, Jiangsu 215123 (CN); LV, Shuang, Suzhou, Jiangsu 215123 (CN); ZHANG, Wei, Suzhou, Jiangsu 215123 (CN)
(74) Representative: Isarpatent
(86) International application number: PCT/CN2022/091873
(87) International publication number: WO 2022/242500

(57) **Abstract**

The invention discloses a method of preparing an antibody carrying a universal site-directed coupling interface based on a genetically modified vertebrate. The present invention provides a method of constructing a vertebrate model, the vertebrate model is used to produce an antibody carrying a universal site-directed coupling interface; the method includes the steps of: site-directly knocking in a coding gene of a specific recognition sequence of a certain ligase A or a coding gene of a certain intein A at a certain position A of a certain coding gene A of the immunoglobulin in the genome of the recipient animal, to obtain the vertebrate model. The modified mice of the present invention can normally stimulate immune responses, produce a polyclonal antibody and a monoclonal antibody with a site-directed linking site. Antibodies with a site-directed linking site can normally carry out site-directed linking, and add various application groups to realize downstream applications of antibodies, such as ELISA, immunofluorescence staining, and immuno-PCR.

## Description

### Technical field

The invention relates to the field of biotechnology, in particular to a method of preparing an antibody carrying a universal site-directed coupling interface based on a genetically modified vertebrate.

### Background of the Invention

Antibodies are protein-based biomacromolecules that bind to corresponding antigens with a strong specificity and affinity. Antibodies are the basis of modern biopharmaceuticals and are widely used in disease treatment, diagnostic reagents and biomedical research. Antibody discovery mainly includes methods such as hybridoma, phage display, and B cell sorting. Vertebrates, such as mice, rabbits, sheep, chickens, and camels, are important animal platforms for the development of monoclonal and polyclonal antibodies.

Previous studies have shown that the introduction of a specific amino acid sequence at a site-directed position of the antibody can achieve a specific linkage reaction mediated by a ligase, such as Sortase, butelase, oaAEP1, and several groups may be site-directly introduced.

So far, there are no reports in the prior art that the antibody-encoding gene is edited directly at the animal level, and the interface-encoding gene is site-directly knocked in, so as to obtain an animal that can directly produce an antibody carrying its own interfaces.

### Summary of the invention

The purpose of the present invention is to provide a method of preparing an antibody carrying a universal site-directed coupling interface based on a genetically modified vertebrate.

In the first aspect, the present invention claims a method of constructing a vertebrate model, the vertebrate model is used to produce an antibody carrying a universal site-directed coupling interface.

The method of constructing a vertebrate model claimed in the present invention may include the steps of: site-directly knocking in a coding gene of a specific recognition sequence of a certain ligase A or a coding gene of a certain intein A at a certain position A of a certain coding gene A of the immunoglobulin in the genome of a recipient animal, to obtain the vertebrate model.

In the method, the coding gene A is the Igkc gene (i.e., the gene encoding the constant region domain of the antibody kappa light chain), and the position A is the 3' end of the Igkc gene. That is, at the antibody level, the specific recognition sequence of the ligase A will be site-directed inserted into the C-terminus of the antibody kappa light chain.

In the method, the vertebrate may be selected from any one of the following: mice, rats, rabbits, sheep, chickens, camels, horses, donkeys, hamsters, guinea pigs, and alpacas.

In a specific embodiment of the present invention, the vertebrate is a mouse; the coding gene A is the Igkc gene on chromosome 6 in the mouse genome; the position A is located between position 70726754 and position 70726755 at the Igkc gene (Gene ID: 16071) on chromosome 6 in the mouse genome (GRcm38/mm10 version of the mouse genome).

In the method, the ligase A may be selected from any of the following: Sortaseₛₜₐₚₕ enzyme, Sortaseₛₜᵣₑₚ enzyme, Butelase enzyme, oaAEP1 enzyme, Formylglycine generating enzyme (FGE), glutamyl transaminase, Tubulin Tyrosine Ligase (TTL), Trypsiligase, Sfp phosphopantetheinyl transferase, and SpyLigase.

When the ligase A is Sortaseₛₜₐₚₕ A (hereinafter collectively referred to as Sortase A) enzyme, the specific recognition sequence may be LPXTG (X is any amino acid); when the ligase A is Sortaseₛₜᵣₑₚ enzyme, the specific recognition sequence may be LPXTA (X is any amino acid); when the ligase A is Butelase enzyme, the specific recognition sequence may be NHV; when the ligase A is oaAEP1 enzyme, the specific recognition sequence may be NGL; when the ligase A is Formylglycine generating enzyme, the specific recognition sequence may be CXPXR (X is any amino acid); when the ligase A is glutamyl transaminase, the specific recognition sequence may be LLQGA; when the ligase A is tubulin tyrosine ligase, the specific recognition sequence may be VDSVEGEEEGEE; when the ligase A is Trypsiligase, the specific recognition sequence may be YRH; when the ligase A is Sfp phosphopantetheinyl transferase, the specific recognition sequence may be DSLEFIASKLA; when the ligase A is Sfp phosphopantetheinyl transferase, the specific recognition sequence may be DSLEFIASKLA; and when the ligase A is SpyLigase, the specific recognition sequence may be AHIVMVDAYKPTK or ATHIKFSKRD.

In the method, the site-directed knock in may be realized by CRISPR/Cas9 technology.

Further, the target sequence cleaved by Cas9 nuclease is located within the range of 500bp upstream and downstream of the position A of the coding gene A of immunoglobulin in the genome of the recipient animal, so as to insert DNA of specific recognition sequence by homologous recombination mechanism.

In a specific embodiment of the present invention, the target sequence is specifically shown in SEQ ID No. 1 (that is, GGAATGAGTGTTAGAGA*CAAAGG, wherein * is the Cas9 cleavage position) (at this time, the vertebrate is a mouse, and the position A of the coding gene A of the immunoglobulin is located at positions 70726754 and 70726755 of the Igkc gene (Gene ID: 16071) on chromosome 6 in the mouse genome (GRcm38/mm10 version of the mouse genome).

Further, the homologous recombination vector used as a site-directed knock in tool contains a DNA fragment A; the DNA fragment A is sequentially composed of the 5' homology arm, the coding gene of the specific recognition sequence of the ligase A or the coding gene of intein A, and 3' homology arm composition. The 5' homology arm is a 120bp sequence located upstream of the position A of the coding gene A of immunoglobulin in the genome of the recipient animal. The 3' homology arm is a 150bp sequence located downstream of the position A of the coding gene A of immunoglobulin in the genome of the recipient animal.

In a specific embodiment of the present invention, the sequence of the 5' homology arm is shown in positions 1-120 of SEQ ID No. 2, and the sequence of the 3' homology arm is shown in positions 157-306 of SEQ ID No. 2.

Further, the coding gene of the specific recognition sequence of the ligase A is shown in positions 133-147 of SEQ ID No. 2 (the ligase A is the Sortase A enzyme, positions 133-147 of SEQ ID No. 2 are the coding gene of the specific recognition sequence of Sortase A enzyme).

Furthermore, the nucleotide sequence of the DNA fragment A is shown in SEQ ID No. 2.

The method of constructing the vertebrate model claimed in the present invention may specifically comprise the steps of:
(1) inject Cas9 mRNA, gRNA and the above-mentioned homologous recombination vector into the fertilized egg cytoplasm of the recipient animal to obtain F0 generation animals;
   The sequence of the gRNA is shown in SEQ ID No. 4.
(2) cross the F0 generation animal obtained in step (1) with the recipient animal, and obtain heterozygous animal, from the F1 generation animal, in which the coding gene of the specific recognition sequence of the ligase A or the coding gene of the intein A is site-directed knocked in at the position A of the coding gene A of the immunoglobulin in the genome A.
   Further, after step (2), the following step (3) may further be included:
(3) cross the male heterozygous animal with the female heterozygous animal one or more times, and from the hybrid offspring obtain a homozygous animal in which the coding gene of the specific recognition sequence of ligase A or the coding gene of intein A is site-directly knocked in at the position A of the coding gene A of the immunoglobulin in the genome.

Both the heterozygous animals in step (2) and the homozygous animals in step (3) may be used as the vertebrate models that may be used to produce antibodies carrying universal site-directed coupling interfaces.

Wherein, the sequence of the Cas9 mRNA may be shown in SEQ ID No. 3.

The homologous recombination vector is specifically the DNA fragment A (SEQ ID No. 2).

In the second aspect, the present invention claims the use of the vertebrate model constructed by the method described in the first aspect above in producing antibodies carrying a universal site-directed coupling interface.

In a third aspect, the present invention claims a method of producing an antibody carrying a universal site-directed coupling interface.

The method of producing an antibody carrying a universal site-directed coupling interface as claimed in the present invention may include the steps of:
P1. prepare a vertebrate model according to the method described in the first aspect above;
P2. immunize the vertebrate model with an immunogen, thereby preparing an antibody carrying a universal site-directed coupling interface and being against the immunogen.

Wherein, the antibody is a monoclonal antibody or a polyclonal antibody.

In a specific embodiment of the present invention, the immunogen is S1 antigen (DongKang Bio, VISC2-S101), and the vertebrate is a mouse (such as a C57BL/6J mouse).

In the fourth aspect, the present invention claims to protect the vertebrate model constructed by using the method described in the first aspect above.

In the fifth aspect, the present invention claims to protect the antibody carrying the universal site-directed coupling interface prepared by the method described in the third aspect above.

In the present invention, the general site-directed coupling interface specifically refers to the specific recognition sequence of the ligase A or the coding gene of the intein A linked to a specific position of the antibody.

When the interface is the specific recognition sequence of the ligase A linked to a specific position of the antibody, the interface and the linker used to link with the effector group (such as HRP, poly-HRP, DNA) may be used to realize the coupling of the described antibody to the effector group, and the linker can catalyze the linkage (such as covalent linkage) of the specific recognition sequence with the linker under the action of the ligase A.

In the present invention, the ligase A is Sortase A enzyme, the specific recognition sequence may be LPXTG (X is any amino acid), and the corresponding linker is a polypeptide molecule whose N-terminal (free end) is an oligoglycine (such as 1-5 consecutive G(s), or 3-5 consecutive G(s) as another example) or a polypeptide molecule whose N-terminus (free end) is an alkylamine.

In a specific embodiment of the present invention, the ligase A is Sortase A enzyme, the specific recognition sequence is LPETG, and the corresponding linker is a polypeptide molecule whose N-terminus (free end) is GGG.

When the interface is the coding gene of the intein A linked to a specific position of the antibody, the antibody is reduced with a reducing agent, and an active group will be generated on it, and the active group may be used to react with an effector molecule or peptide whose initial molecule at N-terminus is cysteine.

Taking mice as an example, the present invention directly introduces a DNA sequence encoding a specific amino acid sequence into the locus of a vertebrate antibody gene, so that the antibody produced by the animal carries a specific site-directed linking site that may be mediated by an enzyme.

### Brief Description of the Drawings

Fig. 1 is a schematic diagram of gene editing in USB mice of the present invention. Schematic diagram of the mouse Igkc locus (genes are shown from left to right, the full length is 532bp), the gray area represents the open reading frame (ORF), and the white area represents the untranslated region (UTR) of the gene. After successful editing, the wild type allele in the genome will be replaced by the mutant allele.
Fig. 2 shows the sequencing results of positive wild type and F0 generation mice. Upper part: gene sequence of wild type mouse; lower part: gene sequence of mutant mouse. The arrow indicates the insertion site of the inserted gene, and gene sequencing of the F0 generation mice showed a mixed sequence after the insertion position, indicating that the insertion was successful.
Fig. 3 shows the genotype detection results of F0 generation mice and F1 mutant mice. A is the genotype of the F0 generation mice detected by PCR, and a total of 6 positive mice (marked numbers) were screened. B is the genotype of F1 mutant mice detected by PCR, and a total of 3 positive mice (marked numbers) were screened.
Fig. 4 shows the genotypes of mutant homozygous mice detected by PCR and sequencing.
Fig. 5 is the detection of the immune effect of the S1 antigen.
Fig. 6 shows the cross-linking reaction of antibodies extracted from the sera of USB heterozygous mice (A) and USB homozygous mice (B) and polypeptides.
Fig. 7 shows the cross-linking reaction between antibodies prepared by culturing hybridoma cells and polypeptides.
Fig. 8 shows the detection result of Sortase A protein with 5-15% SDS-PAGE gradient gel.
Fig. 9 shows the ELISA detection of USB-antibody direct-labeled HRP function.
Fig. 10 shows the result of linking USB-antibody to DNA. A is the sample treated with reducing buffer; B is the sample treated with non-reducing buffer.

### Best Mode of Implementing The present invention

The following examples facilitate a better understanding of the present invention, but do not limit the present invention. The experimental methods in the following examples are conventional methods unless otherwise specified. The test materials used in the following examples, unless otherwise specified, were purchased from conventional biochemical reagent stores. Quantitative experiments in the following examples were all set up to repeat the experiments three times, and the results were averaged.

### Example 1. USB mouse gene editing

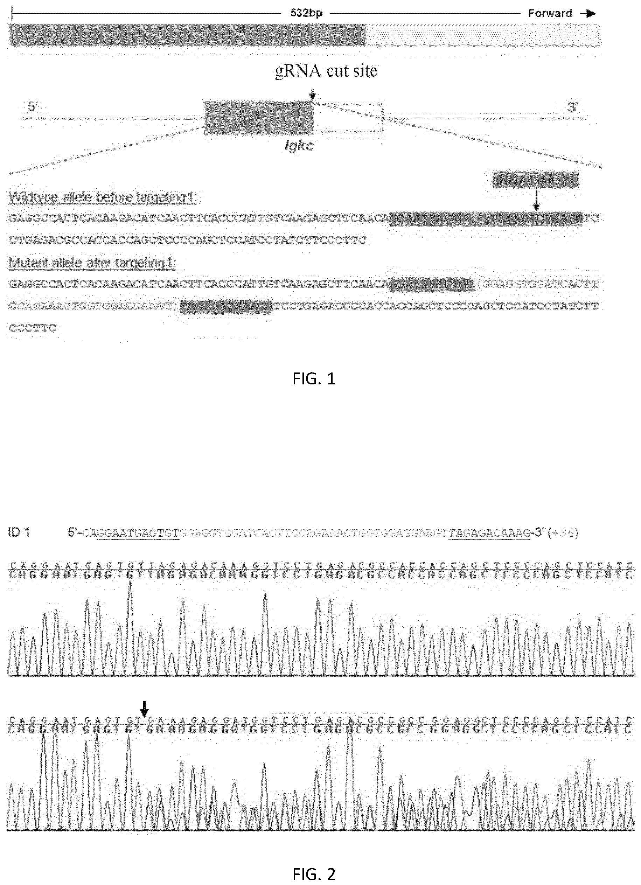

Through CRISPR-Cas9 technology, the sequence of "GGAGGTGGATCACTTCCAGAAACTGGTGGAGGAAGT", that is, "USB" sequence, is inserted between positions 70726754 and 70726755 of the Igkc gene (Gene ID: 16071) of chromosome 6 in the mouse zygote (GRcm38/mm10 version of the mouse genome) through homologous recombination, as shown in Fig. 1. This gene editing makes the amino acid of "GGGSLPETGGGS" is added at the carboxy-terminal of the Igkc light chain of the born mouse, where "LPETG" is the specific recognition site of transpeptidase (Sortase A), which may realize site-directed linkage at the protein level. After embryo implantation, PCR and gene sequencing were carried out, to detect whether there was a "USB sequence" in the born mice. As expected, several born mice had the corresponding correct sequence. Through subsequent mating, pure-line USB mice were generated. Details are provided as follows:

### I. Design of gRNA

The sequence of the gRNA targeting genome of Igkc gene is as follows:
5'-GGAATGAGTGTTAGAGA*CAAAGG-3' (SEQ ID No. 1).

Bases underlined are PAM, and * is the cleavage site.

### II. Homologous recombination vector (donor vector)

Homologous recombination vector (donor vector): DNA chemical synthesis of the DNA fragment shown in SEQ ID No. 2.

Positions 1-120 of SEQ ID No. 2 represent the 5' homology arm; positions 133-147 represent the coding gene of the specific recognition sequence of the Sortase A enzyme, and positions 157-306 shows the 3' homology arm.

### III. In vitro transcription of Cas9 mRNA and gRNA

The nucleotide sequence of Cas9 mRNA obtained by in vitro transcription is shown in SEQ ID No. 3.

The nucleotide sequence of the gRNA obtained by in vitro transcription is shown in SEQ ID No. 4, and its targeting sequence is shown in SEQ ID No. 1.

### IV. Injection of fertilized eggs to obtain F0 chimera mice

Prepare the homologous recombination vector constructed in step 2 and the Cas9 mRNA and gRNA obtained by in vitro transcription in step 3 into a 40 µl system with water, so that the final concentrations of the homologous recombination vector, Cas9 mRNA and gRNA are 100ng/µl and 100ng/µl, and 50ng/µl, respectively. The prepared samples were injected into the fertilized egg cytoplasm of C57BL/6J mice, cultured at 37°C for 24 hours to the two-cell stage, and then transplanted into surrogate female mice (mouse variety ICR), until F0 generation mice were born.

### V. Identification of the genotype of the F0 generation mice

### 1. Extraction of total mouse DNA

(1) Total genomic DNA was extracted using TaKaRa MiniBEST Universal Genomic DNA Extraction kit (Ver.5.0_Code No.9765).
(2) Cut 2-5mm mouse tail tissue, add 180µl GL buffer, 20µl proteinase K and 10µl RNase A to each sample. Incubate overnight at 56°C.
(3) Centrifuge at 12,000 rpm for 2 min to take the supernatant.
(4) Add 200µl GL buffer solution, 200µl absolute ethanol, and mix well.
(5) Add the above mixture to the spin column in the kit, centrifuge at 12,000 rpm for 2 min, and discard the centrifuged liquid.
(6) Add 500µl WA buffer, centrifuge at 12000rpm for 1min, and discard the centrifugation solution.
(7) Add 700µl WB buffer, centrifuge at 12000rpm for 1min, and discard the centrifuged liquid.
(8) Repeat step (7).
(9) Transfer the spin column to a collection tube and centrifuge at 12,000 rpm for 2 min.
(10) Transfer the spin column to a 1.5ml EP tube, add 50-200 µL sterilized water to dissolve the DNA, and place it at room temperature for 5 minutes.

### (11) Centrifuge at 12,000 rpm for 2 min to obtain mouse genomic DNA.

### 2. PCR detection

The PCR reaction mixture was prepared according to the proportions shown in Table 1 below:

**Table 1. PCR reaction mixture preparation ratio**

| Components | Volume |
|---|---|
| Total DNA | 1.5µl |
| 5' primer(10µM) | 1µl |
| 3' primer(10µM) | 1µl |
| dNTP (2.5mM) | 1.5µl |
| 10X PCR buffer (add Mg²⁺) | 3µl |
| TaKaRa Taq HS (5U/µl) | 0.2µl |
| H₂O | 21.8µl |

| | |
|---|---|
| Note: Primer sequence: 5' Primer: 5'-GCTGATGCTGCACCAACTGTATCC-3'; 3' Primer: 5'-GGACTGCCATGTAGTGGACAGCC-3'. | |

PCR product:
Wild type: 695bp
Mutant: 7 3 1 bp

PCR reaction condition setting:
Pre-denaturation 94°C 5min
Denaturation 94°C 30s
Annealing 60°C 30s
Extension 72°C 60s
Terminal extension at 72°C for 5 minutes
Denaturation-annealing-extension total 35 cycles.

The PCR products were sequenced and identified.

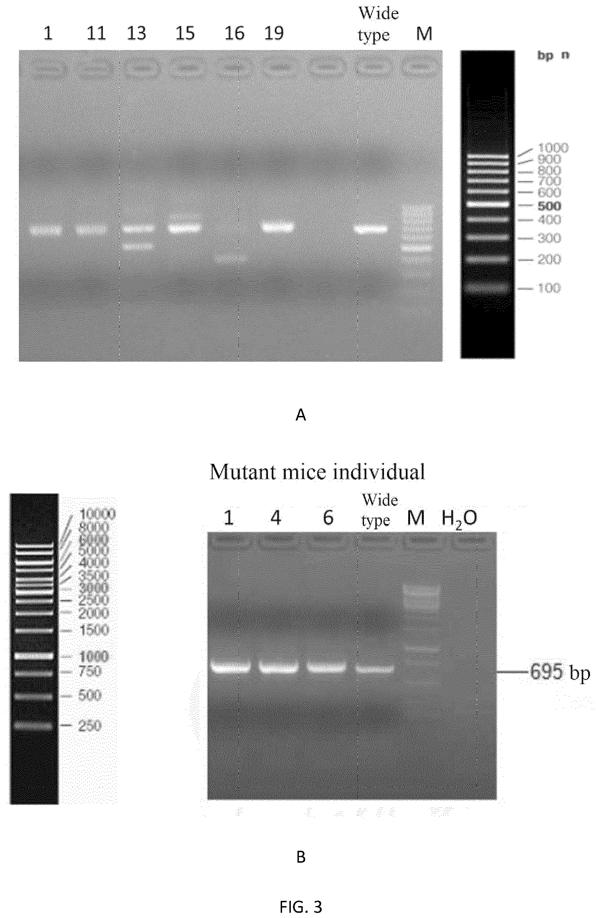

The results show that: through microinjection of fertilized eggs, the present invention obtains 23 F0 generation mice in total. The genotype of the F0 generation mice was identified by PCR+ sequencing, and the PCR results were confirmed by sequencing, and a total of 6 positive F0 generation mice with correct homologous recombination were obtained (Fig. 2 and A in Fig. 3). Fig. 2 shows the sequencing results of wild type and positive F0 generation mice (sequencing primer: GGTGCCTCAGTCGTGTGCTTCTTG).

### VI. Acquisition and genotype identification of F1 generation mice

F0 generation positive mice were mated with wild type C57BL/6J mice to obtain F1 generation positive mice.

The identification steps are the same as F0 (PCR+ sequencing).

The results showed that three mice in the F1 generation were mutant (B in Fig. 3).

### VII. Obtain homozygous positive mice by crossing F1 positive mice

Female F1 positive mice (heterozygous) are mated with male F1 positive mice (heterozygous) to obtain homozygous positive mice from the offspring.

The identification steps are the same as F0 (PCR+ sequencing).

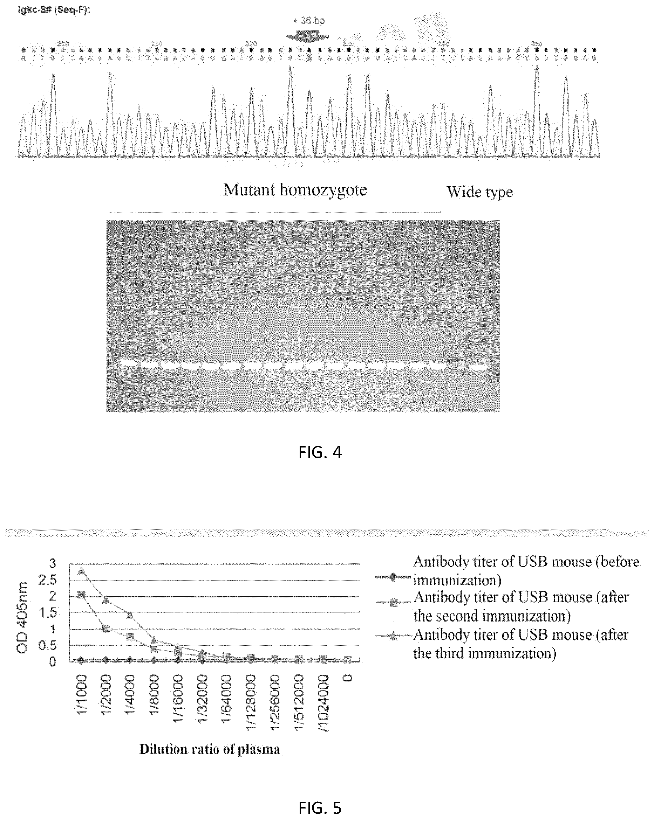

The results showed that all the tested mice were homozygous for the mutant (Fig. 4).

### Example 2. USB mouse immune response

Homozygous USB mice can produce normal immunity should be the premise of using USB mice to produce USB antibodies. Antigen immunization was performed on USB mice (S1 antigen, DongKang Bio, VISC2-S101). Details are provided as follows:
1. Immune animals: take blood from 4-6 weeks of homozygous USB mice (that is, the homozygous positive mice obtained in step VII of Example 1) as a control before immunization. On the first day of the immunization cycle, 30 micrograms of antigen and an equal volume of Freund's complete adjuvant were mixed and injected subcutaneously into mice (Freund's complete adjuvant, Sigma F5881). On day 14, the second immunization (subcutaneous injection of 30 µg antigen and an equal volume of Freund's incomplete adjuvant; Freund's incomplete adjuvant, Thermo, 77140), blood collection on day 21, ELISA detection of serum titer, and the third immunization on day 35 (subcutaneous injection of 30 µg antigen and an equal volume of Freund's incomplete adjuvant), blood collection on day 42 (the third immunization), and ELISA detection of serum titer.
2. ELISA steps:
   (1) Coating solution for S1 antigen (coating solution: dissolve 1.59 g Na₂CO₃ and 2.94 g NaHCO₃ in 990 ml purified water, add 10 ml 10% (mass fraction) NaN₃, pH 9.6. store at 4°C), after dilution, add 100 µl to each well on a 96-well plate, overnight at 4°C.
   (2) Wash with PBS-T for 4 times and block for 1 h.
   (3) Blocking solution for USB mouse serum (PBS-T containing 2% BSA and 0.1% (mass fraction) NaN₃. Stored at 4°C) was diluted 1000, 2000, 4000, 8000, 16000, 32000, 64000, 128000 times, add 100 µl to each well. Incubate at room temperature for 1h.
   (4) Wash with PBS-T for 4 times.
   (5) Dilute the HRP-labeled rat anti-mouse secondary antibody (Aibosheng, 030604A06H) 1:1000 with PBST, and add 100 µl of chromogenic solution to each well. Incubate at room temperature for 1h.
   (6) Wash with PBS-T for 4 times.
   (7) Prepare chromogenic solution: dissolve 4 mg ABTS (azino-di-3-ethyl-benzthiazodinsulphonate) in 10 ml of 50 mM citric acid, pH 4.0, add 10 µl 30% H₂O₂. Add 100 µl chromogenic solution to each well.
   (8) Read the value at a wavelength of 405 nm after color development.

Serum was taken before immunization, after the second immunization, and after the third immunization, and the titer of the serum was detected by ELISA. The results are shown in Table 2 and Fig. 5. It can be seen that the antibody titer of the USB mouse gradually increased, which means that it has a normal immune response.

**Table 2. Antibody titers of USB mice gradually increased with immunization**

| | 1/100 0 | 1/200 0 | 1/400 0 | 1/800 0 | 1/1600 0 | 1/3200 0 | 1/6400 0 | 1/12800 0 | 1/25600 0 | 1/5120 0 0 | 1/1024 000 | 0 |
|---|---|---|---|---|---|---|---|---|---|---|---|---|
| Antibody titer of USB mouse (before immunization ) | 0.063 | 0.065 | 0.071 | 0.058 | 0.066 | 0.059 | 0.064 | 0.062 | 0.076 | 0.059 | 0.084 | 0.06 |
| Antibody titer of USB mouse (after the second immunization ) | 2.054 | 1.015 | 0.767 | 0.402 | 0.286 | 0.163 | 0.156 | 0.121 | 0.091 | 0.074 | 0.08 | 0.06 7 |
| Antibody titer of USB mouse (after the third immunization ) | 2.792 | 1.905 | 1.446 | 0.681 | 0.481 | 0.292 | 0.117 | 0.102 | 0.09 | 0.081 | 0.067 | 0.06 1 |

### Example 3. Verification of USB interface function

### I. Linkage reaction of polyclonal antibody extracted from mouse serum under USB genetic background with polypeptide

In order to verify whether the antibody secreted by B cells produced in the USB mouse may be catalyzed by the enzyme, the polypeptide was site-directed linked to the light chain region containing the USB sequence. This example uses antibodies extracted from serum of USB heterozygous (that is, the F1 generation heterozygous positive mice obtained in Example 1) and USB homozygous (that is, the F1 generation homozygous positive mice obtained in Example 1) mouse to react with the polypeptide containing the recognition sequence of Sortase A, and the linkage product was detected by Western blot.

### 1. Experimental materials

Sera from USB heterozygous mice and USB homozygous mice identified in Example 1; protein A beads (Sigma, 5015979001), Sortase A enzyme (see Example 4 for specific preparation methods), polypeptide (GGGSYPYDVPDYAGKPIPNPLLGLDS TEQKLISEEDLK, Beijing Zhong Ke Yaguang Biotechnology Co., Ltd.), PBS, HRP-labeled rat anti-mouse secondary antibody (Aibosheng, 030604A06H), ECL luminescent liquid (Shanghai Tianneng, 180-5001).

### 2. Experimental steps

(1) Take 20 µl of serum from USB heterozygous mice and USB homozygous mice respectively, and use 30 µl protein A beads to extract total antibodies to detect the concentration of purified antibodies.
(2) Mix the peptide (GGGSYPYDVPDYAGKPIPNPLLGLDSTEQKLISEEDLK) with the antibody at a 50-fold molar concentration, and add Sortase A enzyme to a final concentration of 2µM. The negative control is the peptide (GGGSYPY DVPDYAGKPIPNPLLGLDSTEQKLISEEDLK) and antibody mixture, without Sortase A enzyme, overnight at 37°C.
(3) Take 15µl of the reaction mixture, reduce the sample on 12% gel, transfer to the membrane for 3 h, and block with 5% skimmed milk powder overnight.
(4) Dilute HRP-labeled rat anti-mouse IgG secondary antibody (Aibosheng, 030604A06H) in milk + PBST at 1:3000 (volume ratio), room temperature for 1 h, and develop color.

### 3. Experimental results

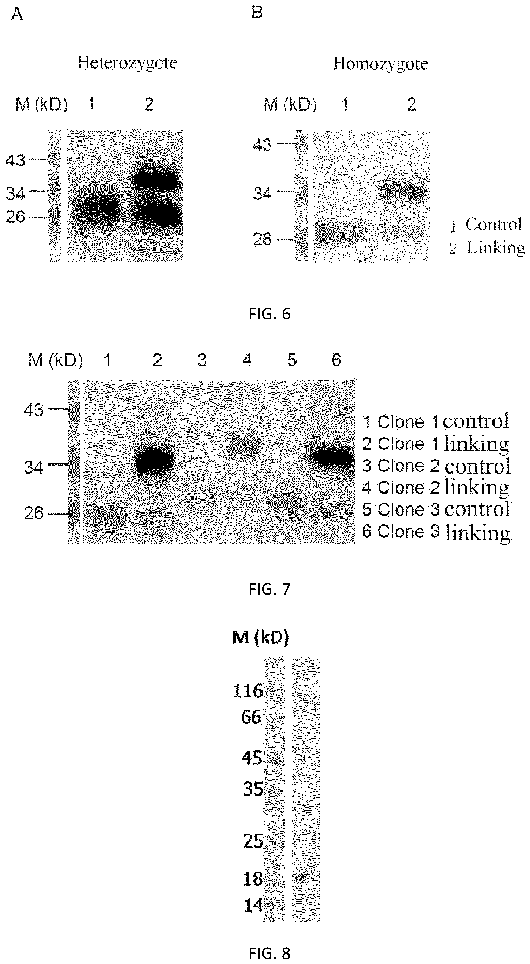

After the naturally produced antibody in the USB heterozygous mouse is cross-linked with the polypeptide, the molecular weight becomes larger. In the linkage group, the band that appears above the prototype kappa chain is the linkage product, and this band does not appear in the control group (A in Fig. 6). Since not all cells of heterozygous mice carry the USB gene, the secreted antibodies are a mixture of normal antibodies and antibodies carrying the USB interface. After linking with the polypeptide, a considerable part of the antibodies still fail to link to the polypeptide.

In contrast, almost all antibodies produced in USB homozygous mice carry USB tags, and after cross-linking with polypeptides, most of the antibodies can cross-link with polypeptides (B in Fig. 6).

This experiment proves that the antibody in mice under USB genetic background can complete the cross-linking reaction under the catalysis of Sortase A enzyme.

### II. Linkage reaction of monoclonal antibody produced by bone marrow hybridoma cells under USB genetic background with polypeptides

Bone marrow hybridoma is an important source of raw materials for large-scale production of antibodies. In this example, bone marrow hybridoma cells were prepared from B cells extracted from homozygous USB mice (i.e., the homozygous positive mice of the F1 generation obtained in Example 1), and monoclonal antibodies were extracted from the cell culture supernatant, and the monoclonal antibodies were verified to cross-link polypeptides to antibody light chains with Sortase A enzyme.

### 1. Experimental materials

Bone marrow hybridoma cells (prepared from B cells extracted from USB mice obtained in Example 1); transpeptidase Sortase A; protein A adsorption beads (Sigma, 5015979001); polypeptide (GGGSYPYDVPDYAGKPIPNPLLGLDS TEQKLISEEDLK, Beijing Zhongkeya Synthesized by Photobiotech Co., Ltd.); HRP-labeled rat anti-mouse secondary antibody (Aibosheng, 030604A06H);

### 2. Experimental steps

(1) Preparation of hybridoma cells: Cell fusion may be carried out after the serum antibody titer reaches 1:8000. S1 antigen (DongKang Biological, VISC2-S101) was injected intravenously 3 days before fusion. On the day of fusion, mouse splenocytes were fused with Sp2/0 cells, and the hybridoma cells generated by fusion were grown in HAT selective medium (Sigma, H0262).
(2) Screen cell lines with higher antibody titers: ELISA detection. The method is the same as above. As for the first few concentrations of the hybridoma cell supernatant doubling diluted, the ELISA reading is higher than 1.8, and the higher the antibody titer, the better.
   During the detection, it was coated with S1 antigen (DongKang Bio, VISC2-S101), and the culture supernatant of hybridoma cells was used as the primary antibody, and HRP-labeled rat anti-mouse IgG (Aibosheng, 030604A06H) was used as the secondary antibody. Cell lines stably expressing specific antibodies were expanded and frozen.
(3) Take 3ml of supernatant, use 100µl protein A adsorption beads to extract antibody, and detect the concentration of purified antibody.
(4) The setting and operation of the antibody-peptide cross-linking reaction experimental group and the control group are the same as steps 1 and 2.

### 3. Experimental results

After the three selected monoclonal antibodies were linked to the polypeptide, the light chains all shifted up (Fig. 7). This experiment proves that the monoclonal antibody carrying the USB tag prepared by culturing hybridoma cells in vitro can cross-link specific molecules to the antibody under the action of Sortase A enzyme.

### Example 4. practical use of USB antibody

### I. Use of monoclonal antibody produced by USB mouse in ELISA

### (1) Experimental materials

1. S1 antigen, DongKang Bio, VISC2-S101;
2. Preparation of Sortase A protein

### Sequence:

ATGCAGGCAAAACCGCAGATTCCGAAAGATAAAAGCAAAGTGGCCGG
TTATATTGAAATTCCGGATGCAGATATTAAGGAACCGGTGTATCCGGGCCC
GGCAACCCGCGAACAGCTGAATCGTGGCGTGAGTTTTGCCAAAGAAAATC
AGAGTCTGGATGATCAGAATATTAGCATTGCAGGTCATACCTTTATTGATC
GTCCGAATTATCAGTTTACCAATCTGAAAGCAGCCAAAAAAGGTAGCATG
GTTTATTTTAAAGTGGGCAATGAAACCCGTAAATATAAAATGACCAGTATT
CGTAACGTGAAACCGACCGCAGTTGAAGTGCTGGATGAACAGAAAGGCAA
AGATAAACAGCTGACCCTGATTACCTGTGATGATTATAATGAAGAAACCG
GTGTGTGGGAAACCCGTAAGATTTTTGTGGCCACCGAAGTGAAACTCGAG
CACCACCACCACCACCACTGA(SEQ ID No. 5)

(1) Ndel and XhoI recognition sequences were constructed at the 5' and 3' ends of the Sortase A gene, respectively, and inserted into the corresponding restriction sites of the pET30a(+) vector, and the recombinant plasmid pET30a-Sortase A was obtained after sequencing and verification. The recombinant plasmid pET30a-Sortase A was transformed into competent BL21(DE3), and cultured in Kan⁺ resistant solid LB medium for 16 h.
(2) Pick a monoclonal colony and culture it in LB medium (Kan⁺ resistant) until the bacterial OD₆₀₀ is 0.6, and add IPTG at 30°C to induce expression for 4 h.
(3) The expressed bacteria were ultrasonically lysed, and the supernatant before induction and after induction is lysed and precipitated, and detected by SDS-PAGE. The results showed that the Sortase A protein can be expressed in a soluble form.
(4) Expand the culture of bacteria, and induce the expression by IPTG at 30°C for 4 h.
(5) Sonicate the bacteria, and purify the lysed supernatant through a Ni column.

Finally, the purity of Sortase A protein detected by SDS-PAGE is greater than 90%. Sortase A protein was detected by 5-15% SDS-PAGE gradient gel (Fig. 8).
3. USB mice: the F1 generation homozygous positive mice obtained in Example 1 and the F1 generation heterozygous positive mice obtained in Example 1.
4. Polypeptide-biotin, sequence: GGGSENLYFQAK-biotin, synthesized by Zhongke Yaguang.
5. PD-10 purification column, GE Healthcare, 17085101.
6. Streptavidin-HRP, Sigma, S5512.
7. HRP-labeled rat anti-mouse secondary antibody (Aibosheng, 030604A06H)

### (II) Experimental method

1. Immunization of animals: On the first day, mix 30 micrograms of S1 antigen and an equal volume of Freund's complete adjuvant, and inject 4-6 week-old USB mice subcutaneously at multiple points. On day 21, 42, and 63, the booster immunization was performed using 30 micrograms of antigen mixed with an equal volume of Freund's incomplete adjuvant, and the mice were injected subcutaneously at multiple points. On day 1, 28, 49, and 70, blood was collected from the tail vein, and the serum titer was detected by ELISA (the serum collected on the first day was used to detect the antibody titer of the pre-immune serum). Antibody titer is defined as the reciprocal of the maximum dilution factor at which the serum sample reads is greater 0.5 than the background.
2. Fusion cell screening: Cell fusion may be performed after the serum antibody titer reaches 1:8000. S1 antigen was injected intravenously three days before fusion. On the day of fusion, mouse splenocytes were fused with Sp2/0 cells, and the hybridoma cells generated by fusion were grown in HAT selective medium.
3. Screening cell lines with high antibody titers: ELISA is used to detect whether the hybridomas secrete specific antibodies. During the detection, the antigen (S1 antigen) was coated, and the hybridoma cell culture supernatant was used as the primary antibody, and HRP-labeled rat anti-mouse IgG (Aibosheng, 030604A06H) was used as the secondary antibody, and the cell line stably expressing the specific antibody (denoted as S1-UBS antibody) was screened, expanded culture and cryopreserved.
4. Extract the S1-UBS antibody, mix the antibody with 50-fold molar concentration of peptide-biotin, and add Sortase A protein to a final concentration of 2 µM. Linkage overnight at 37°C. The linkage product is antibody-polypeptide-biotin.
5. Antibody-polypeptide-biotin was purified by PD-10 purification column.
6. Take equimolar amounts of Streptavidin-HRP and antibody-polypeptide-biotin to incubate, so as to obtain Antibody-HRP.
7. Set up the control, that is, the mixture of the antibody with the S1-USB interface and Streptavidin-HRP. Because the S1-USB antibody is not linked to the polypeptide-biotin, it cannot theoretically link with Streptavidin-HRP and cannot develop color.
8. Antibody-HRP and control group were used for ELISA. The steps of ELISA are as follows:
   (1) Dilute the S1 antigen with coating solution to 0.1-5g/ml, add 100µl to each well on a 96-well plate, and store in a humid chamber at 4°C overnight.
   (2) Wash with PBS-T for 4 times.
   (3) Add 160-200 µl of blocking solution to each well, and incubate for 1 h at room temperature in a humid chamber.
   (4) Dilute the primary antibody (the "antibody-HRP" obtained in step 6 or the control in step 7) in PBS-T or blocking solution, add 100 µl to each well. Incubate in a humid chamber at room temperature for 1 h.
   (5) Wash with PBS-T for 4 times.
   (8) Add 100 µl of chromogenic solution to each well.
   (9) Read the value at a wavelength of 405 nm after color development.

### (III) Experimental results

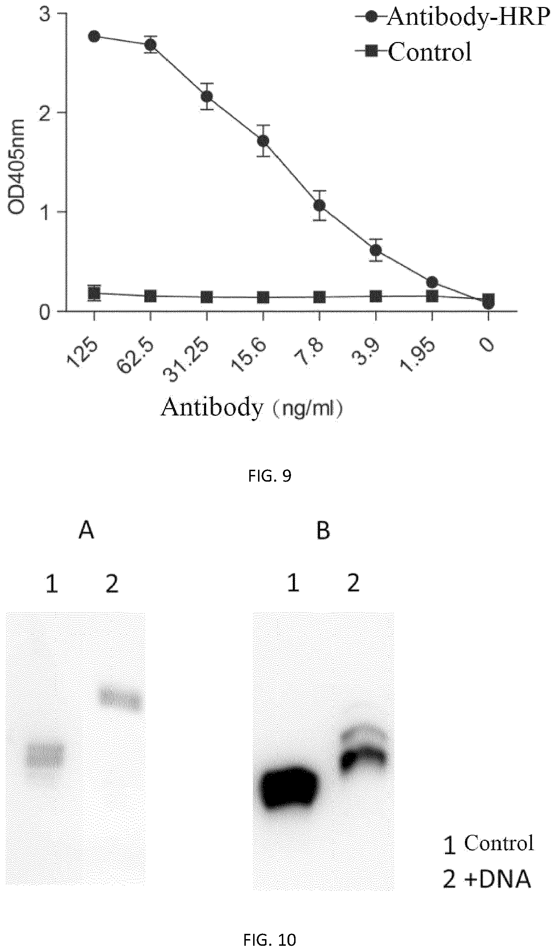

The result is shown in Fig. 9. It can be seen that the hybridoma cells obtained by the fusion of B cells immunized with USB mice and myeloma cells can secrete antibodies containing UBS interfaces, which may be catalyzed by Sortase A to carry biotin groups and combine with streptavidin-HRP. The S1 antibody carrying HRP is formed, and the S1 - HRP antibody has the ability to normally recognize the antigen and develop color. In contrast, the S1-USB antibody cannot bind to streptavidin-HRP in the absence of biotin, resulting in very low background. It further explains the key role of the USB interface in this model.

### II. Application of polyclonal antibody produced by USB mice in linkage with DNA

### (I) Experimental materials:

1. S1 antigen, DongKang Bio, VISC2-S101;
2. The preparation of Sortase A protein is the same as step I.
4. Polypeptide-azide: GGGSYPYDVPDYAGKPIPNPLLGLDSTEQKLISE EDLK-azide (C-terminal azide modification), synthesized in Zhongke Yaguang.

### 3. DNA oligos

Sequence: 5'-CAGGTAGTAGTACGTCTGTTTCACGATGAGACTGGATTCG-3', DBCO modification at the 5' end; Synthesized in Anhui General.

### (II) Experimental method

1. The method of immunizing animals with S1 antigen is the same as step 1.
2. After the immunization process is over, kill the mice, take 2ml of serum from the immunized mice, incubate with 500µl protein A beads and serum for 1 h, wash the protein A beads with 1ml PBS for 3 times, wash the protein A beads with 0.1ml pH 2.0 glycine buffer for 5 minutes, the pH of the eluent was adjusted to 7.4 with 2M Tris-HCl pH 8.0 to obtain polyclonal antibodies against S1 protein. Quantification was performed using a BCA kit (Thermofisher, 23225).
3. Mix 100 µg of polyclonal antibody with 25-fold molar concentration of polypeptide-azide, and add Sortase A protein to a final concentration of 2 µM. Linkage overnight at 37°C. The linkage product is antibody-polypeptide-azide.
4. The antibody-polypeptide-azide is purified with PD-10 purification column. Quantification was performed using a BCA kit (Thermofisher, 23225).
5. Take 2 µg of polyclonal antibody and incubate with 2-fold molar concentration of DNA oligo, and react overnight at room temperature.
   The control was a sample incubated with the same amount of antibody and water.
6. The reaction mixture was mixed with reducing loading buffer and non-reducing loading buffer respectively, and boiled for 5 minutes.
7. Detection of mouse kappa light chain by Western blot. The antibodies used were rat anti-mouse kappa light chain antibody (Aibosheng Biotechnology Co., Ltd., 116111C10), and HRP-labeled mouse anti-rat secondary antibody (Aibosheng Biotechnology Co., Ltd., 011003G07H).

### (III) Experimental results

The results are shown in Fig. 10. Compared with the control, the position of the light chain of the antibody treated with the reducing buffer was obviously shifted up (A in Fig. 10). Non-reducing buffer treatment showed that the molecular weight of the whole antibody also shifted up (B in Fig. 10). These results showed that the antibody produced by USB mice was catalyzed by Sortase A to carry azide group, and the antibody was linked with DBCO-DNA oligo to generate antibody-DNA complexes, which may be used for relevant detection.

The present invention has been described in detail above. For those skilled in the art, without departing from the spirit and scope of the present invention, and without unnecessary experiments, the present invention may be practiced in a wider range under equivalent parameters, concentrations and conditions. While specific embodiments of the invention have been shown, it should be understood that the invention may be further modified. In summary, according to the principles of the present invention, this application intends to include any changes, uses or improvements to the present invention, including changes made by using conventional techniques known in the art and departing from the disclosed scope of this application. Applications of some of the essential features are possible within the scope of the appended claims below.

### Industrial application

Mouse antibodies are composed of heavy chains and light chains, and more than 95% of antibody light chains are kappa chains. This invention adds a specific amino acid sequence to the C-terminal of the kappa chain through CRISPR-mediated gene knock in, making more than 95% of antibodies produced by genetically modified mice carry specific site-directed linking sites that may be mediated by enzymes. The site-directed linking site introduced in the example of the present invention is the recognition site of Sortase A. Experiments show that the modified mice can normally stimulate immune responses and produce polyclonal antibodies and monoclonal antibodies with site-directed linking sites. The produced antibodies with site-directed linking sites may be site-directly linked normally, and various application groups may be added to realize downstream applications of antibodies, such as ELISA, immunofluorescent staining, and immuno-PCR.

## Claims

1. A method of constructing a vertebrate model, the vertebrate model is used to produce an antibody carrying a universal site-directed coupling interface; the method comprises the steps of: a coding gene of a specific recognition sequence of a certain ligase A or a coding gene of a certain intein A is site-directly knocked in at a certain position A of a certain coding gene A of an immunoglobulin in the genome of a recipient animal, to obtain the vertebrate model.

2. The method according to claim 1, **characterized in that**: the coding gene A is the Igkc gene, and the position A is the 3' end of the Igkc gene.

3. The method according to claim 1, **characterized in that**: the vertebrate is selected from any one of the following: mice, rats, rabbits, sheep, chickens, camels, horses, donkeys, hamsters, guinea pigs, and alpacas.

4. The method according to claim 3, **characterized in that**: the vertebrate is a mouse; the coding gene A is the Igkc gene on chromosome 6 in the mouse genome, and the position A is located between positions 70726754 and 70726755 of the Igkc gene on chromosome 6 in the mouse genome.

5. The method according to any one of claims 1-4, **characterized in that**: the ligase A may be selected from any of the following: Sortaseₛₜₐₚₕ enzyme, Sortaseₛₜᵣₑₚ enzyme, Butelase enzyme, oaAEP1 enzyme, Formylglycine generating enzyme, glutamine acyltransaminase, Tubulin Tyrosine Ligase, Trypsiligase, Sfp phosphopantetheinyl transferase, and SpyLigase.

6. The method according to claim 5, **characterized in that**: when the ligase A is Sortaseₛₜₐₚₕ A enzyme, the specific recognition sequence is LPXTG, and X is any amino acid;
when the ligase A is Sortaseₛₜᵣₑₚ enzyme, the specific recognition sequence is LPXTA, and X is any amino acid;
when the ligase A is Butelase enzyme, the specific recognition sequence is NHV;
when the ligase A is oaAEP1 enzyme, the specific recognition sequence is NGL;
when the ligase A is a Formylglycine generating enzyme, the specific recognition sequence is CXPXR, and X is any amino acid;
when the ligase A is glutamyl transaminase, the specific recognition sequence is LLQGA;
when the ligase A is tubulin tyrosine ligase, the specific recognition sequence is VDSVEGEEEGEE;
when the ligase A is Trypsiligase, the specific recognition sequence is YRH;
when the ligase A is Sfp phosphopantetheinyl transferase, the specific recognition sequence is DSLEFIASKLA;
when the ligase A is Sfp phosphopantetheinyl transferase, the specific recognition sequence is DSLEFIASKLA; and
when the ligase A is SpyLigase, the specific recognition sequence is AHIVMVDAYKPTK or ATHIKFSKRD.

7. The method according to any one of claims 1-6, **characterized in that**: the site-directed knock in is realized by using CRISPR/Cas9 technology.

8. The method according to claim 7, **characterized in that**: the target sequence cleaved by the Cas9 nuclease is located within 500bp range upstream and downstream of the position A of the coding gene A of immunoglobulin in the genome of the recipient animal.

9. The method according to claim 8, **characterized in that**: the target sequence is shown in SEQ ID No. 1.

10. The method according to claim 9, **characterized in that**: the homologous recombination vector serving as a site-directed knock in tool contains a DNA fragment A; the DNA fragment A is sequentially composed of 5' homology arm, the coding gene of the specific recognition sequence of ligase A or the coding gene of the intein A, and the 3' homology arm; the 5' homology arm is a 120bp sequence located upstream of the position A of the coding gene A of the immunoglobulin in the genome of the recipient animal; the 3' homology arm is a 150bp sequence located downstream of the position A of the coding gene A of the immunoglobulin in the genome of the recipient animal.

11. The method according to claim 10, **characterized in that**: the 5' homology arm is shown in positions 1-120 of SEQ ID No. 2.

12. The method according to claim 10, **characterized in that**: the 3' homology arm is shown in positions 157-306 of SEQ ID No. 2.

13. The method according to claim 10, **characterized in that**: the coding gene of the specific recognition sequence of the ligase A is shown in positions 133-147 of SEQ ID No. 2.

14. The method according to any one of claims 10-13, **characterized in that**: the nucleotide sequence of the DNA fragment A is shown in SEQ ID No. 2.

15. The method according to any one of claims 1-14, **characterized in that**: the method comprises the steps of:
(1) inject Cas9 mRNA, gRNA and the homologous recombination vector according to any one of claims 10-14 into the fertilized egg cytoplasm of the recipient animal to obtain F0 generation animals; and
the sequence of the gRNA is shown in SEQ ID No. 4;
(2) cross the F0 generation animals obtained in step (1) with the recipient animal, and obtain heterozygous animal, from the F1 generation animal, in which the coding gene of the specific recognition sequence of the ligase A or the coding gene of the intein A is site-directed knocked in at the position A of the coding gene A of the immunoglobulin in the genome A.

16. The method according to claim 15, **characterized in that**: after step (2), the following step (3) is further included:
(3) cross the male heterozygous animal with the female heterozygous animal one or more times, and from the hybrid offspring obtain a homozygous animal in which the coding gene of the specific recognition sequence of ligase A or the coding gene of intein A is site-directly knocked in at the position A of the coding gene A of the immunoglobulin in the genome.

17. The method according to claim 15 or 16, **characterized in that**: the sequence of the Cas9 mRNA is shown in SEQ ID No. 3.

18. The use of the vertebrate model constructed by the method of any one of claims 1-17 in the production of antibodies carrying universal site-directed coupling interfaces.

19. A method of producing an antibody carrying a universal site-directed coupling interface, comprising the steps of:
P1. prepare a vertebrate model according to the method according to any one of claims 1-17;
P2. immunize the vertebrate model with an immunogen, thereby preparing an antibody carrying a universal site-directed coupling interface and being against the immunogen.

20. The method according to claim 19, **characterized in that**: the antibody is a monoclonal antibody or a polyclonal antibody.

21. A vertebrate animal model constructed by the method of any one of claims 1-17.

22. An antibody carrying a universal site-directed coupling interface prepared by the method of claim 19 or 20.
